# EUROPÄISCHE PATENTSCHRIFT

(11) **EP 2 661 629 B1**
(45) Veröffentlichungstag und Bekanntmachung des Hinweises auf die Patenterteilung: **07.03.2018**
(21) Anmeldenummer: 12701461.1
(22) Anmeldetag: 09.01.2012
(51) Int. Cl.: G01N 33/574, G01N 33/68, G01N 33/50

(54) **VERFAHREN ZUR DIAGNOSE DER FAMILIÄREN ADENOMATÖSEN POLYPOSIS (FAP)**
METHOD FOR DIAGNOSING FAMILIAL ANDENOMATOUS POLYPOSIS (FAP)
PROCÉDÉ DE DIAGNOSTIC DE LA POLYPOSE ADÉNOMATEUSE FAMILIALE (PAF)

(30) Priorität: 07.01.2011 DE 102011008050
(43) Veröffentlichungstag der Anmeldung: 13.11.2013
(73) Patentinhaber: Universität zu Lübeck, 23538 Lübeck (DE)
(72) Erfinder: GEMOLL, Timo, 23775 Großenbrode (DE); ROBLICK, Uwe, Johannes, 23552 Lübeck (DE); AUER, Gert, 17160 Solna (SE); HABERMANN, Jens, Karsten, 29227 Celle (DE)
(74) Vertreter: Steinecke, Peter
(86) Internationale Anmeldenummer: PCT/EP2012/050258
(87) Internationale Veröffentlichungsnummer: WO 2012/093178

(56) Entgegenhaltungen:
- EP-A1- 2 028 492
- US-A1- 2008 213 816
- YEUNG ANTHONY T ET AL: "One-hit effects in cancer: Altered proteome of morphologically normal colon crypts in Familial Adenomatous Polyposis", CANCER RESEARCH, Bd. 68, Nr. 18, 15. September 2008 (2008-09-15), Seiten 7579-7586, XP002669847, ISSN: 0008-5472 -& YEUNG ANTHONY T ET AL: "Supplementary Data:One-hit effects in cancer: Altered proteome of morphologically normal colon crypts in Familial Adenomatous Polyposis", CANCER RESEARCH, 15. September 2008 (2008-09-15), Seite 29PP, XP002670920,
- ROBLICK U J ET AL: "Sequential proteome alterations during genesis and progression of colon cancer.", CELLULAR AND MOLECULAR LIFE SCIENCES : CMLS, Bd. 61, Nr. 10, Mai 2004 (2004-05), Seiten 1246-1255, XP002669848, ISSN: 1420-682X
- FRANCIS F. LAM ET AL: "Identification of distinctive protein expression patterns in colorectal adenoma", PROTEOMICS - CLINICAL APPLICATIONS, Bd. 4, Nr. 1, 1. Januar 2010 (2010-01-01), Seiten 60-70, XP055019699, ISSN: 1862-8346, DOI: 10.1002/prca.200900084

## Beschreibung

Die Erfindung betrifft ein Verfahren zur Diagnose der familiären adenomatösen Polyposis (FAP).

Die dritthäufigste Ursache von in der westlichen Welt auftretenden tumorassoziierten Todesfällen ist der Darmkrebs, wobei über 85-95 % aller kolorektalen Karzinome spontan auftreten. Bei den verbleibenden 5-15 % handelt es sich um vererbte Formen, unter ihnen die dominant vererbte familiäre adenomatöse Polyposis (FAP).

Statistisch gesehen betrifft die familiäre adenomatöse Polyposis etwa 2,3-3,2 von 100.000 Menschen. In den letzten Jahrzehnten wurden etwa 0,5 % aller kolorektalen Karzinome als durch FAP verursacht diagnostiziert. Glücklicherweise ist diese Zahl aufgrund verbesserter Methoden zur Diagnose von FAP rückläufig, sodass Risikopatienten frühzeitig erkannt und einer prophylaktischen totalen Kolektomie unterzogen werden können.

Typischerweise entwickeln FAP-Patienten zwischen ihrem 10. und 30. Lebensjahr hunderte bis tausende von Kolon-Adenomen oder adenomatösen Polypen. Im Alter von 35 weisen etwa 95 % der betroffenen Personen Polypen auf. Polypen werden zwar als grundsätzlich gutartig, jedoch im Hinblick auf die familiäre adenomatöse Polyposis auch als prämaligne Läsionen während der kolorektalen Karzinogenese angesehen. Ohne prophylaktische Kolektomie führt die große Anzahl dieser prämalignen adenomatösen Läsionen praktisch zum Fortschreiten von invasiv wachsendem Krebs.

Die familiäre adenomatöse Polyposis ist eine autosomal, dominant vererbte Veranlagung, die auf Mutationen des APC-Gens im Chromosom 5q21 zurückzuführen ist. Abhängig von der Position der Mutation im APC-Gen, kann das FAP-Syndrom unterschiedlich ausgeprägt sein und FAP-Patienten mögen eine abgeschwächte Form der Krankheit mit nur einer relativ kleinen Anzahl von Polypen entwickeln.

Die am häufigsten auftretende Mutation im APC-Gen liegt bei Codon 1309. Mutationen in diesem Codon führen bereits in einem sehr frühen Alter zu einer hohen Anzahl von Kolon-Adenomen. Als Ursache hierfür wird diskutiert, dass das APC-Gen normalerweise als "Gatekeeper" fungiert, jedoch bei Auftreten von Mutationen fehlerhaft agiert und so zu einer Deregulierung der Apoptose, einem Ungleichgewicht des Zellerneuerungsvorgangs und damit zur Ausbildung des Polyposis-Syndroms führt.

Zur Diagnose einer Person mit Verdacht auf FAP wird hauptsächlich auf die Anamnese, körperliche Untersuchungen und genetische Tests zurückgegriffen. Insbesondere werden genetische Untersuchungen immer dann durchgeführt, wenn die klinischen Befunde zur sicheren Diagnose von FAP nicht ausreichend sind.

Allerdings können auch die genetischen Untersuchungen mit den derzeit zur Verfügung stehenden Tests nicht ein eindeutiges Ergebnis liefern, sodass in einigen Fällen auch ein (falsch) negatives Ergebnis derartiger molekularer Tests nicht ausschließen kann, dass der Patient nicht doch FAP-Träger sein könnte und die typischen klinischen Symptome erst zu einem späteren Zeitpunkt entwickelt.

Daher werden oftmals auch weitere Untersuchungen, beispielsweise der Netzhaut der betroffenen Patienten und dessen Familienangehörigen, unternommen, die jedoch nur unzureichend Auskunft über die genetische Disposition des Patienten geben können.

In einer Veröffentlichung von Yeung et al., Cancer Research 68 (2008), 7579-7586 werden die Proteine KRT18 und ECH1 als unterschiedlich regulierte Proteine zwischen unauffälliger Darmschleimhaut von Patienten mit FAP und unauffälliger Darmschleimhaut von Patienten mit einem kolorektalen Karzinom beschrieben.

Aufgabe der Erfindung ist es daher ein alternatives Verfahren zur Diagnostik, einschließlich weiterer Biomarker, insbesondere zur Früherkennung der familiären adenomatösen Polyposis (FAP) bereitzustellen.

Diese Aufgabe wird durch das Verfahren mit den Merkmalen von Anspruch 1 gelöst. Die Unteransprüche geben vorteilhafte Ausgestaltungen der Erfindung wieder.

Dementsprechend betrifft die Erfindung ein Verfahren zum Diagnostizieren der familiären adenomatösen Polyposis (FAP), gekennzeichnet durch die Schritte: Bestimmen der Expression wenigstens eines Proteins ausgewählt aus der Gruppe bestehend aus RNF115, HSPA8, HSPD1, ASTN2, KRT9, KRT10, UQCRC1, CCT5, NDUFS1, ACTB, LMNA, ACY1, RUVBL1, CSTF2T, TIGD5, ECH1, ALDH2 und KRT18 in einer Gewebeprobe eines ersten Individuums; und Vergleichen der Expression des wenigstens einem Proteins in der Gewebeprobe des ersten Individuums mit der Expression des wenigstens einen Proteins in einer Gewebeprobe eines gesunden zweiten Individuums und/oder eines dritten Individuums mit sporadisch kolorektalem Karzinom, wobei das Vorliegen einer erhöhten Expression des wenigstens einen Proteins in der Gewebeprobe des ersten Individuums das Vorliegen der familiären adenomatösen Polyposis (FAP) anzeigt und wobei die Gewebeprobe eine Probe der Darmschleimhaut ist. Die vollen Proteinnamen sind den Tabellen 2a bis 2c zu entnehmen.

Das vorstehend beschriebene Verfahren ist weiterhin dadurch gekennzeichnet, dass das Vorliegen einer erhöhten Expression von ECH1 in der Gewebeprobe des ersten Individuums das Vorliegen der familiären adenomatösen Polyposis (FAP) in einem Polypen und/oder Krebszellen aufweisenden Stadium anzeigt.

Das vorstehend beschriebene Verfahren ist zusätzlich dadurch gekennzeichnet, dass das Vorliegen einer erhöhten Expression von ALDH2 und/oder KRT18 in der Gewebeprobe des ersten Individuums das Vorliegen der familiären adenomatösen Polyposis (FAP) in einem Krebszellen aufweisenden Stadium anzeigt.

In einer bevorzugten Ausführungsform sind die vorstehend beschriebenen Verfahren dadurch gekennzeichnet, dass zusätzlich die Expression von HNF4A und/oder TNF in der Gewebeprobe des ersten Individuums bestimmt und mit der Expression von HNF4A und/oder TNF in der Gewebeprobe des zweiten Individuums und/oder der Gewebeprobe des dritten Individuums verglichen wird, wobei das Vorliegen einer erhöhten Expression von HNF4A und/oder TNF in der Gewebeprobe des ersten Individuums das Vorliegen der familiären adenomatösen Polyposis (FAP) anzeigt.

Ferner betrifft die vorliegende Erfindung die Verwendung eines Kits in einem der vorstehend beschriebenen erfindungsgemäßen Verfahren, wobei das Kit Antikörper umfasst, die spezifisch für den Nachweis von mindestens einem Protein ausgewählt aus einer Gruppe bestehend aus RNF115, HSPA8, HSPD1, ASTN2, KRT9, KRT10, UQCRC1, CCT5, NDUFS1, ACTB, LMNA, ACY1, RUVBL1, CSTF2T, TIGD5, ECH1, ALDH2, KRT18 und gegebenenfalls FAP, HNF4A und/oder TNF sind.

Darüber hinaus betrifft die vorliegende Erfindung die Verwendung einer multiplexen Testplatte in einem der vorstehend beschriebenen erfindungsgemäßen Verfahren, auf der Antikörper aufgebracht sind, die spezifisch den Nachweis von mindestens einem Protein erlauben ausgewählt aus einer Gruppe bestehend aus RNF115, HSPA8, HSPD1, ASTN2, KRT9, KRT10, UQCRC1, CCT5, NDUFS1, ACTB, LMNA, ACY1, RUVBL1, CSTF2T, TIGD5, ECH1, ALDH2, KRT18 und gegebenenfalls FAP, HNF4A und/oder TNF. Bevorzugt ist die multiplexe Testplatte eine Chip-Oberfläche und/oder ein ELISA.

Grundgedanke der Erfindung ist es, das Proteinexpressionsprofil einer Gewebeprobe eines Patienten mit Verdacht auf familiäre adenomatöse Polyposis (FAP) zu bestimmen und somit die Erkrankung bereits in einem Frühstadium, in dem noch keine klinischen Anzeichen zu finden sind, zu diagnostizieren.

Bevorzugt kann das Proteinexpressionsprofil der Gewebeprobe des Patienten mit Verdacht auf familiäre adenomatöse Polyposis (FAP) auch mit dem Proteinexpressionsprofil einer Gewebeprobe eines an sporadisch auftretendem Darmkrebs erkrankten Patienten verglichen werden, um das Ergebnis gegenüber dieser Krankheitsform abzugrenzen.

Mittels multivariater statistischer Analyse, einschließlich t-Test, exaktem Test nach Fisher, a-posteriori-Wahrscheinlichkeit und hierarchischer Clusteranalyse, konnten spezifische Proteine und Proteinmuster auf allen Krankheitsebenen identifiziert werden, die für diagnostische Zwecke verwendet werden können.

Bevorzugt wird für die Gewebeprobe aus der Darmschleimhaut entnommenes Biopsiematerial gewonnen, wobei dieser Eingriff routinemäßig und ohne größeren Aufwand vorgenommen werden kann.

Mittels 2-D-Gel-Elektrophorese wurde von 47 Proben, die von 15 verschiedenen Patienten erhalten wurden (FAP, n=8; sporadischer kolorektaler Krebs, n=7), ein Proteinprofil angefertigt. Die Analyse wurde für normale Schleimhaut, Adenome und Karzinome durchgeführt, während FAP-assoziiertes Gewebe mit dem sporadischen Gegenstück verglichen wurde. Ein Spot-Vergleich versuchte, Proteine zu identifizieren, die in FAP-Gewebe exprimiert wurden, aber nicht in dem entsprechenden sporadischen Gewebe oder umgekehrt, wobei insbesondere FAP-assoziierte normale Schleimhaut mit sporadischer normaler Schleimhaut verglichen wurde. Proteine wurden durch Massenspektrometrie identifiziert, einer Ingenuity Pathway-Analyse unterzogen, und Zielproteine wurden durch Western Blot validiert.

In allen makroskopisch unberührten FAP-Schleimhautproben lagen insgesamt 47 Proteine vor, fehlten aber in sporadischer normaler Schleimhaut. Ein Vergleich von FAP-Polypen mit sporadischen Dickdarmpolypen offenbarte 49 Polypeptide, die in den FAP-Proben vorlagen, aber in allen sporadischen Polypen fehlten. Nur ein Protein zeigte ein entgegengesetztes Expressionsmuster mit hoher Expression in sporadischen Polypen. Ein Vergleich von drei FAP-Karzinomen mit sieben sporadischen kolorektalen Karzinomen ergab 66 Proteine mit einem Abwesenheits-/Anwesenheitsexpressionsmuster. Von diesen konnten 46 Proteine durch Massenspektrometrie identifiziert werden und wurden einer Ingenuity Pathway Analyse (IPA) unterzogen. HSPD1, CSTF2T, RUVBL und ACTB wurden exemplarisch durch Western Blot signifikant validiert (p<0,05), während KRT10 die Signifikanz marginal verfehlte (p = 0,0553).

Die Erfindung wird im Folgenden, auch anhand der in den Zeichnungen dargestellten Figuren, näher erläutert. Es zeigen:
- Fig. 1: ein Mengendiagramm der qualitativ unterschiedlich exprimierten Proteine bei FAP und sporadischen Krankheitsverläufen. Es ist dabei zu beachten, dass der Vergleich von normal FAP zu normal sporadisch (n=47), Polyp FAP zu Polyp sporadisch (n=50) und Krebs FAP zu sporadisch auftretendem Krebs (n=66) Proteine aufweist, die jeweils eine Teilmenge bilden. Daher beträgt die Anzahl die einen Gesundheitszustand eindeutig charakterisieren 44 für normal, 37 für Polyp und 53 für Krebs.
- Fig. 2: eine Heatmap von 47 unterschiedlich exprimierten Proteinen aus normal FAP und normal sporadischer Mucosa.
- Fig. 3: das IPA-Netzwerk 1 von 13 Proteinen, die unterschiedlich exprimiert sind in makroskopisch nicht betroffener "normaler" FAP-Mucosa und normalen Mucosazellen von Patienten mit sporadisch kolorektalem Krebs. Rot/dunkelgrau markierte Proteine sind in der FAP Mucosa hochreguliert.
- Fig. 4: eine Heatmap von 49 unterschiedliche exprimierten Proteinen zwischen FAP Polypen und sporadischen Polypen.
- Fig. 5: das IPA Netzwerk 1 (Treffer 30) von 12 Proteinen, die unterschiedlich exprimiert sind in FAP Polypen und sporadischen Polypen. Rot/dunkelgrau markierte Proteine sind bei FAP Polypen hochreguliert.
- Fig. 6: das IPA Netzwerk 2 (Treffer 25) von 11 Proteinen, die unterschiedlich exprimiert sind in FAP Polypen und sporadischen Polypen. Rot/dunkelgrau markierte Proteine sind bei FAP Polypen hochreguliert.
- Fig. 7: das IPA Netwerk 1 (Treffer 12) von vier Proteinen, die unterschiedlich im FAP Karzinom und sporadischen Karzinom exprimiert sind. Rot/dunkelgrau markierte Proteine sind im FAP Karzinom hochreguliert.
- Fig. 8: eine Western Blot Analyse, die bestätigt, dass die ausgewählten Proteinen HSPD1, KRT10, CSTF2T, RUVBL und ACTB signifikant unterschiedlich in normaler Mukosa und in sporadischen und FAP Patienten exprimiert sind. Zu beachten ist, dass der p-Wert für KRT10 leicht größer ist als 0,05 (p=0,0553).

### BEISPIELE

### Material und Methoden

### Probenentnahme

Darmproben von Patienten mit "sporadischem" Karzinom und von Patienten mit FAP wurden unmittelbar nach der Resektion von der Chirurgieabteilung, Universitätsklinikum Schleswig-Holstein, Campus Lübeck, und der Chirurgieabteilung, Universität Düsseldorf, gemäß der Richtlinien der jeweiligen lokalen Ethikbehörde erhalten. Zellen wurden von nichtnekrotischen Tumorbereichen, normaler Mucosa und Polypen gemäß einer bekannten Technik gesammelt. Angereicherte Zellen der verschiedenen (Sub-) Entitäten wurden in 2 - 5 ml eiskaltem Medium RPMI-1640 verdünnt, das 5 % Kälberserum und 0,2 mM Phenylmethylsulfonylfluorid / 0,83 mM Benzamidin enthielt. Aspirations- und Spritzschritte mit einer Spritze und einer 29er Nadel halfen bevorzugt solche Zellen freizusetzen, die die geringste Bindung an das Bindegewebe haben (normale Kolonozyten, Adenom- und Tumorzellen). Mit einem Filtersystem (Porengrößen 250 µm und 160 µm) wurden übrige Bindegewebekomponenten eingefangen und ausschließlich der Durchtritt für die Zellen gestattet, die vor der Untersuchung angereichert werden sollten. Diese mit 2 ml eiskaltem Percoll (54,7 % in PBS) unterlegten Zellsuspensionen wurden 10 Minuten lang bei +4 °C bei 400 g zentrifugiert. Die Zellen wurden gesammelt und mit kalter PBS mit einem pH-Wert von 7,4 gewaschen. Dieses Verfahren führte zu reinen Zellpopulationen. Die Validität des Verfahrens wurde früher evaluiert. Die Qualität jeder Probe wurde geprüft, wobei Giemsa-gefärbte Abstriche mit den entsprechenden histologischen Schnittpräparaten verglichen wurden. In die Evaluation wurden nur Proben berücksichtigt, bei denen mehr als 95 % der Zellen angereichert waren. Zellextrakte wurden dann wie beschrieben für eine 2-D-Gelelektrophorese vorbereitet.

### Charakterisierung von Formalin-fixierten Proben

Die histopathologische Charakterisierung erfolgte mit Hämatoxylin-Eosin-gefärbten Schnitten von mit Formalin fixierten und in Paraffin eingebetteten Proben. Polypen und Tumore wurden entsprechen ihrer Größe, Lymphknotenstatus und Ort der Metastasierung klassifiziert. Die histopathologische Klassifizierung wurde basierend auf den WHO-Kriterien durchgeführt (TNM-Klassifikation).

### Protein-Quantifizierung

Die Proteinkonzentration der Proben wurde durch Hinzufügen von 25 µl konzentriertem Assayreagenz (Bio-Rad) zu 1 µl solubilisierter und in 100 µl Milli-Q Wasser gelöster Probe unter Verwendung von 96-Loch-Mikrotiterplatten bestimmt. Eine Standardkurve wurde unter Verwendung verschiedener Konzentrationen von Rinderserum-Albumin angefertigt (Multiscan reader; Labsystems).

### Polypeptidtrennung durch isoelektrische Fokussierung in der ersten Dimension

2-D-Gelelektrophorese wurde wie zuvor beschrieben durchgeführt. Alle Proben wurden in 500 µl 7 M Harnstoff, 2 M Thioharnstoff, 1 % 3-(3-chloramidopropyl)dimethylammonio-1-propansulfonat (CHAPS), 0,4 % immobilisierter pH-Gradienten (IPG)-Puffer, 0,3 % Dithiothreitol (DTT) und einer Spur Bromphenolblau verdünnt. Zellsuspensionen wurden über aktive Rehydrierung auf vorgefertigten IPG-Streifen (Bio-Rad pH 4-7, 17 cm) aufgetragen. Das Protein wurde geladen und in einem PROTEAN IEF Gerät (Bio-Rad) bei ∼52.900 Vh über Nacht fokussiert. Nach der IEF wurden die Streifen sofort für 2 x15 min mit 50 mM Tris-HCl, pH 8,8, in 6M Harnstoff, 30 % Glycerin und 2 % SDS äquilibriert. DTT (1 %) wurde im ersten und Iodacetamid (2,5 %) im zweiten Äquilibrierungsschritt zum Reduzieren und Alkylieren der freien Thiole hinzugefügt.

### Polypeptidtrennung in der zweiten Dimension mittels SDS/PAGE

SDS-Gele mit einem linearen Gradienten von 10 bis 13 % Acrylamid (1,5 x 200 x 230 mm) wurden für die zweite Dimension verwendet. Die IEF-Streifen wurden auf der Oberseite des Gels gelegt und mit warmer Agarose (0,5 %) fixiert. Elektrophorese wurde über Nacht bei 42.000 Vh durchgeführt. Für die Bildanalyse wurden die Gele mit Silbernitrat gefärbt. Spots der präparativen Gele wurden unter Verwendung der Sypro Ruby Färbung sichtbar gemacht.

### Scannen und Bildanalyse

2-D-Gels wurden mit einem GS-710 Densitometer (BioRad) bei einer Auflösung von 84,7 x 84,7 Mikrometern gescannt. Mit der Software PDQuest 8.0 (BioRad) wurden die Daten analysiert. Ein "Match-Satz" mit 47 Gelen wurde konstruiert. Mit der Software PDQuest 8.0 wurden Polypeptid-Spots von verschiedenen Stadien von beiden Krankheiten Spots in dem Standardgel zugeordnet. Die Spotintensitäten wurden normiert, der Hintergrund wurde subtrahiert, Proteinspots wurden lokalisiert und die relativen Färbungsdichten wurden bestimmt. Verschiedene bekannte Proteine dienten als Landmarken. Jeder Spot erhielt eine eindeutige Identifikationsnummer. Alle differentiell exprimierten Spots wurden individuell untersucht, um sicherzustellen, dass sie korrekt zugeordnet, repräsentativ und von hoher Qualität waren. Gesättigte Spots wurden von der Analyse ausgeschlossen.

### Statistische Analyse

Kandidatenmarker wurden durch eine statistische Analyse identifiziert, die sich auf Expressionsdifferenzen konzentrierte, wobei der t-Test, der exakte Test nach Fisher und die a-posteriori-Wahrscheinlichkeitsanalyse kombiniert mit hierarchischem Clustern verwendet wurden. Daten wurden in einem Excel-Datenformat exportiert, das die Proteinmessungen für alle Proben enthielt, verknüpft mit Proben-ID und -Typ (d. h. FAP normal, Polyp, Krebs / sporadisch normal, Polyp, Krebs). Fehlende Werte wurden als -1 kodiert. Zur Analyse von Western-Blot-Daten wurden einseitige t-Tests mit alternativen Hypothesen auf der Basis von beobachten Expressionsdifferenzen in 2-DE-Geldaten berechnet.

### In-Gel-Verdauung

Eine enzymatische Verdauung wurde wie zuvor beschrieben an den entfernten Spots durchgeführt. Gelstücke wurden entfärbt. Trypsin (25 µl einer 12 ng/µl-Lösung in 50 mM Ammoniumbicarbonat) wurde zugesetzt und die Inkubation wurde 4,5 Stunden lang bei 40°C ausgeführt. Peptide wurde mit 30 µl 5 % Ameisensäure / 2 % Acetonitril extrahiert, gefolgt von Extraktion mit 24 µl 2,5 %Ameisensäure / 50 % Acetonitril. Das Acetonitril wurde über Nacht bei 10°C unter Atmosphärendruck verdampft. Zur Elektrosprayionisierung - (ESI) MS/MS - wurden die Peptidextrakte mit C18 ZipTips (Millipore) entsalzt, wurden aktiviert und mit 10 µl 70 % Acetonitril / 0,1 % Trifluoressigsäure (TFA) zweimal, 10 µl 50 % Acetonitril / 0,1 % TFA zweimal und schließlich 10 µl 0,1 % TFA zweimal äquilibriert. Die Probe wurde durch zwanzigmaliges Pipettieren auf die ZipTip geladen und zweimal mit 10 µl 0,1 % TFA gewaschen. Die tryptischen Fragmente wurden mit 60 % Acetonitril / 1 % Essigsäure eluiert.

### Massenspektrometrie (MS)

Die tryptischen Fragmente wurden in einem Ultraflex TOF/TOF-Instrument (Bruker Daltonics, Billerica, MA, USA) analysiert. Proben wurden mit einer gesättigten α-Cyano-4-Hydroxy-Zimtsäure-Lösung in 50 % Acetonitril / 0,1% TFA bei einem Verhältnis 1:1 (v/v) gemischt. Datenbanksuchen wurden mit dem Softwarepaket ProFound (http://prowl.rockefeller.edu) in der nicht-redundanten Protein-Datenbank des National Center for Biotechnology Information (NCBI) ausgeführt. "Mammalia" wurde für die taxonomische Kategorie gewählt. Alle Peptidmassen wurden als monoisotop und [M+H]+ (protonierte Molekülionen) angenommen. Die Suchparameter beinhalteten die Carbamidomethylierung von Cystein durch Iodacetamid als feste Modifikation und Oxidation von Methionin als variable Modifikation. Eine verpasste Trypsinspaltung wurde gestattet. Es wurden nur Proteintreffer mit drei oder mehr passenden Peptinmassen und einer Sequenzabdeckung von mindestens 10 % in Betracht gezogen. Eine interne Kalibrierung wurde durch eine Analyse von autolytischen Trypsin-Spaltungsprodukten erreicht, was zu einer Genauigkeit ±0,05 Da führte. Die Beurteilung der Signifikanz basierte auf dem Erwartungswert, der Anzahl von passenden Peptinmassen und einer Übereinstimmung zwischen experimentellen und theoretischen physikalischen Eigenschaften der Proteine.

### Pfadanalyse

Als exploratorischer Ansatz wurde die Software von Ingenuity Pathways Analysis (IPA) (Ingenuity, MountainView, CA, USA; www.ingenuity.com) verwendet, um die Beteiligung von signifikant differentiell exprimierten Proteinen in bekannten Pfaden und Netzwerken zu beurteilen. IPA ist eine weit verbreitete umfangreiche Datenbank und Software auf der Basis der Ingenuity Pathways Knowledge Base (IPKB). Der Term "Pfad" wird verwendet, um sich auf kanonische Pfade zu beziehen, die relevante Proteine bekannten metabolischen oder Signalisierungskaskaden zuordnen, wie in der Kyoto Encyclopedia of Genes and Genomes (KEGG, www.genome.jp/kegg) beschrieben. Netzwerke werden jedoch durch die IPA dynamisch als Gruppen von Proteinen generiert, die mit einem definierten relevanten Protein interagieren, das heißt, in unserer Analyse den differentiell exprimierten Proteinen.

IPA-Netzwerke können mehrere Proteine enthalten und auch die Identifikation von indirekten Interaktionen zwischen relevanten Targets gestatten. Somit werden die hochgeladenen relevanten Proteine zum Abbilden von molekularen Netzwerken verwendet, die anzeigen, wie diese einander beeinflussen können, und zwar über jene bereits bekannten Interaktionskarten hinaus, wie beispielsweise aus KEGG-Pfaden abgeleitet. Die IPA-generierten Netzwerke werden nach Punktwerten aufgeführt, wobei das obere Netzwerk mit dem höchsten Punktwert eine niedrige Wahrscheinlichkeit besitzt, dass die Generierung der Netze eine glückliche war. Dieser Punktwert spiegelt den negativen Logarithmus des P-Werts wider, der die Wahrscheinlichkeit angibt, dass die relevanten Proteine aufgrund einer Zufallschance zusammen in einem gemeinsamen Netzwerk gefunden werden. Ein Punktwert größer oder gleich "3" wird als ein gültiges Netzwerk angesehen.

### Western Blot

Ausgewählte Proteine auf der Basis der 2-DE-Versuche und Pfadanalyse wurden mit kommerziell erhältlichen Antikörpern weiter validiert. Insgesamt 15 µg Zelllysat wurde durch Elektrophorese auf einem 7,5 % oder 16 % SDS-Polyacrylamid-Gel (150V) in einem laufenden Gelpuffer aufgelöst, der 25 mM Tris, pH-Wert 8,3, 162 mM Glycin und 0,1 % SDS enthielt. Die Proben wurden 80 Minuten lang bei 150 mA auf PVDF-Membranen übertragen.

Um den Proteintransfer zu steuern, wurde jede Membran reversibel mit Ponceau-Rot gefärbt. Die Membranen wurden dann 60 Minuten lang in 5 % Trockenmilch blockiert und über Nacht für folgende Antikörper inkubiert (1 µg/ml): HSPD1 monoklonaler Antikörper (AM03128PU-N, Acris-Antikörper, Herford, Deutschland), KRT10 monoklonaler Antikörper (ab1421, Abcam, Cambridge, UK), CSTF2T polyklonaler Antikörper (H00023283-B01, Abnova, Taipei, Taiwan), RUVBL monoklonaler Antikörper (HPA019947, Sigma, St. Louis, MO, USA), LMNA monoklonaler Antikörper (HPA006660, Sigma, St. Louis, MO, USA), und ACTB monoklonaler Antikörper (ab6276, Abcam, Cambridge, UK). Nach der Inkubation der Membranen mit Ziege-Anti-maus-Sekundärantikörper (1:2000, Santa Cruz Biotech, Santa Cruz, CA, USA) wurde die finale Visualisierung mit dem ECL-Kit (Bio-Rad) durchgeführt. Eine densitometrische Analyse wurde mit QuantityOne-Software Version 4.5.2 (Bio-Rad, CA, USA) ausgeführt.

### Histopathologische Ergebnisse

Klinische Patientendaten und eine histopathologische Diagnose werden in Tabelle 1 bereitgestellt. Alle Darmkrebsproben wurden als Adenokarzinome klassifiziert. Die in der Studie enthaltenen acht "sporadischen" Polypen waren gestielte röhrenförmige Adenome. Alle Polypen hatten eine "Kopf"-Größe mit einem Durchmesser kleiner als 0,5 cm. Das normale Schleimhautgewebe wurde von makroskopisch unbeeinflussten Gebieten in Resektionsproben von beiden gesammelt, FAP- und auch sporadische Fälle.

### Untersuchung der Proteinexpression in sporadischer und FAP-normaler Schleimhaut, Polypen und Tumorgewebe

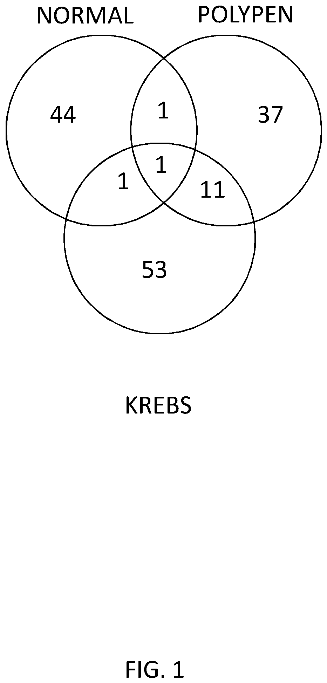

Proteinmessungen wurden für 1950 separierte Proteine aus insgesamt 47 Proben erhalten, die von 15 verschiedenen Patienten gesammelt wurden (8x FAP; 7x sporadisches Karzinom). Um die ausgeprägtesten Unterschiede bei der Proteinexpression für eine potentielle diagnostische Verwendung zu detektieren, wurde in erster Linie versucht, Spots zu bestimmen, die nur bei einer Krankheitsstadiumsgruppe detektiert werden konnten (zum Beispiel FAP- oder sporadisches Gewebe), aber nicht in der anderen, und umgekehrt. Diese Analyse ergibt 47, 50 und 66 Proteinspots, die in FAP-normalem, Polypen- bzw. Tumorgewebe differentiell exprimiert sind, im Vergleich zu den gleichen Krankheitsstadien des sporadischen Gegenstücks (Fig. 1). Individuelle Krankheitsstadiumsvergleiche zwischen FAP- und sporadischem Gewebe sind unten ausführlich beschrieben:

### Makroskopisch unbeeinflusste "normale" FAP-Mucosaproteinsignatur

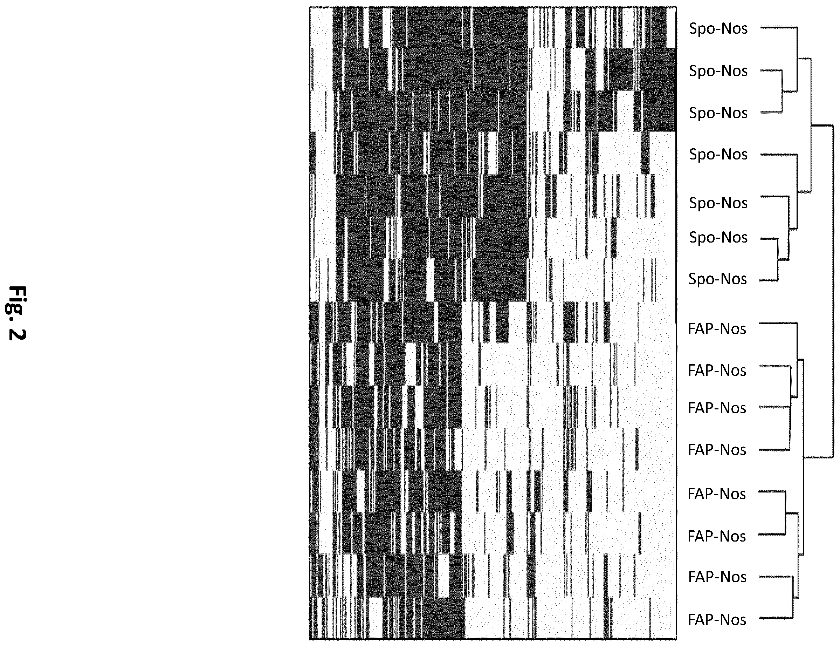

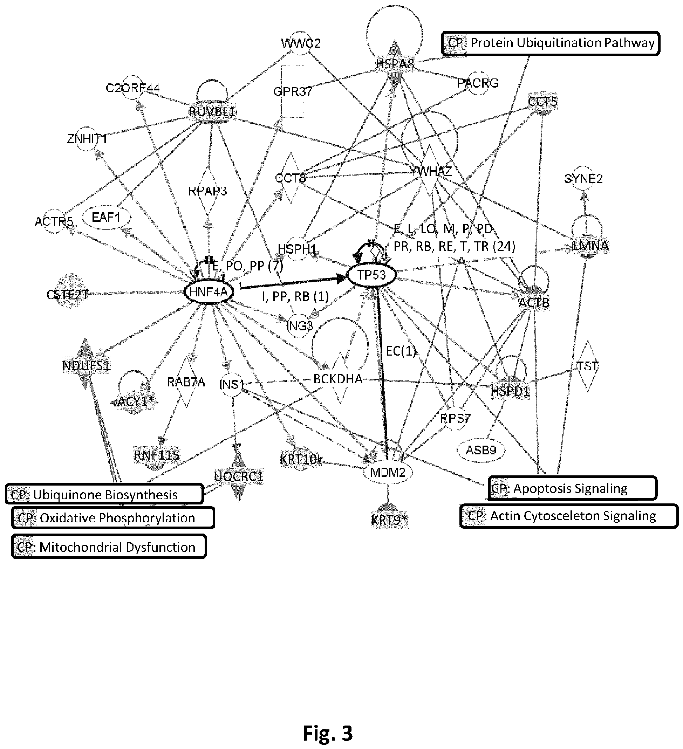

Ingesamt 47 Proteine waren ausschließlich in der normalen FAP-Mucosa anwesend und fehlten in der sporadischen normalen Mucosa. Drei dieser Proteine wurden auch beim Vergleich von FAP und sporadischem Gewebe von Polypen beziehungsweise Krebs gefunden (Fig. 1). Die differentielle Expression der anwesenden/abwesenden Daten ist in Fig. 2 als eine Heatmap visualisiert. Von den 47 Protein-Spots wurden 15 individuelle Proteine durch Massenspektrometrie identifiziert und in die Ingenuity Pathway Analysis (IPA) hochgeladen (Tabelle 2a). Insgesamt 13 Proteine kamen für die IPA-Analyse in Frage, die alle in einem einzigen Netzwerk geclustert waren. Dieses Netzwerk erreichte einen hohen Punktwert von 37 und ist mit Funktionen hinsichtlich Zelltod, dermatologischen Krankheiten und Konditionen und Zellfunktion und -erhaltung assoziiert. Die meisten der 13 Proteine sind direkt mit dem Transkriptionsregulator HNF4A und dem Turmorunterdrücker TP53 verbunden und/oder werden davon reguliert (Fig. 3).

### Polypproteinsignatur

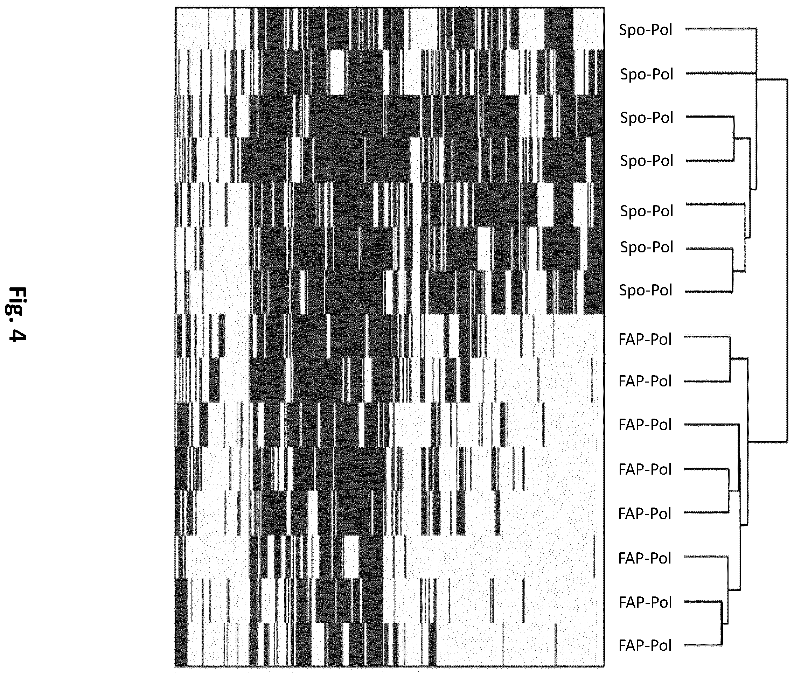

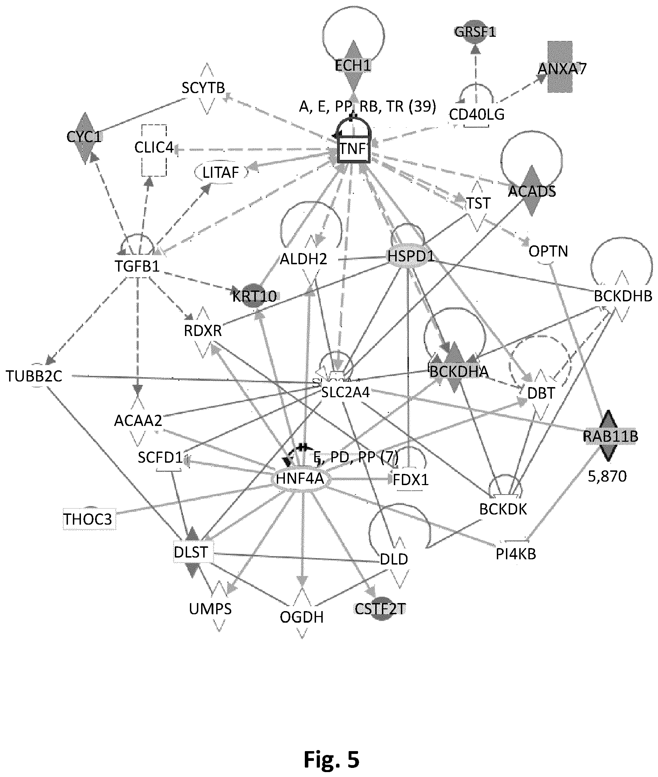

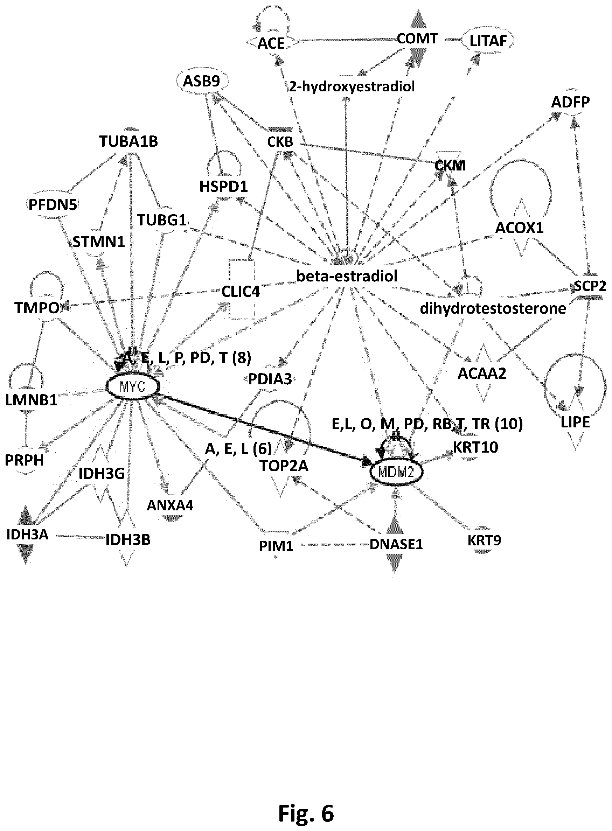

49 Polypeptide wurden aufgefunden, die in den FAP-Polypproben anwesend sind wurden aufgefunden, aber in allen sporadischen Polypen fehlten. Drei dieser Proteine wurden auch in normaler FAP-Schleimhaut exprimiert und ein Protein auch in einem FAP-Karzinom. Fig. 4 visualisiert die Heatmap der differentiellen Proteinexpression. Unter diesen 49 Protein-Spots wurden 23 individuelle Proteine durch Massenspektrometrie identifiziert und zu Ingenuity Pathway Analysis (IPA) hochgeladen (Tabelle 2b). Alle 23 Proteine kamen für eine IPA-Analyse in Frage. Sie clusterten in drei verschiedenen Netzwerken: Netzwerk 1 umfasste 12 relevante Proteine mit einem Punktwert von 30 und war mit Funktionen hinsichtlich genetischen Störungen, metabolischer Krankheit und Energieproduktion assoziiert (Fig. 5). Netzwerk 2 hatte einen Punktwert von 25 und erhielt 11 differentiell exprimierte Proteine bezüglich Zellzyklus, genetischer Störung, DNA-Replikation, Rekombination und Reparatur (Fig. 6). Netzwerk 3 zeigte einen Punktwert von 2 und enthielt nur ein differentiell exprimiertes Protein (HCCS) und wurde als von niedrigerer Signifikanz angesehen. Zentrale Knoten von Netzwerk 1 sind TNF und HNF4A, wohingegen wir für Netzwerk 2 MYC und MDM2 als wichtige Knoten sahen.

### Karzinomproteinsignatur

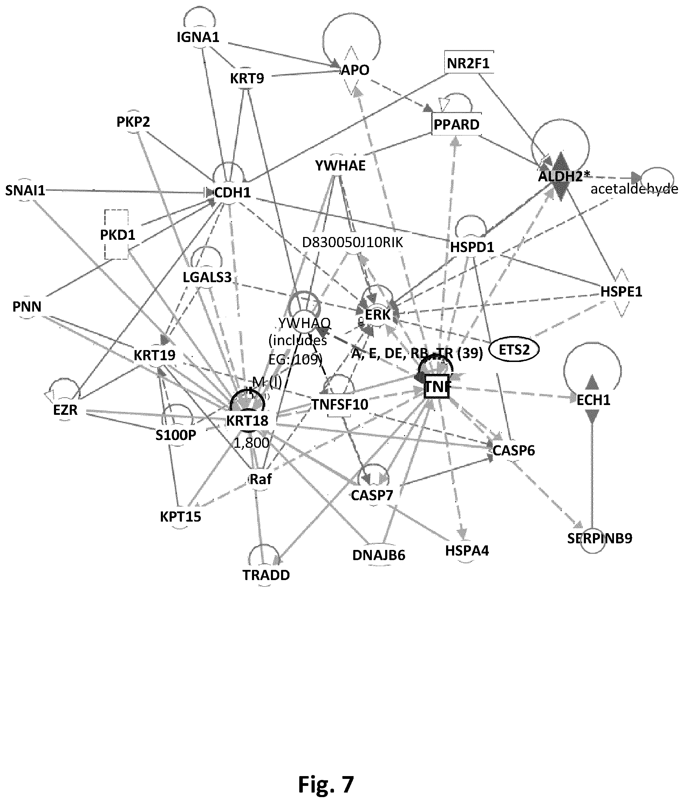

Es standen drei FAP-Karzinomfälle zur Verfügung. Trotz dieser niedrigen Probenanzahl wurde darauf ausgerichtet, potentielle Differenzen bei der Proteinexpression zwischen FAP-assoziierten und sporadischen Karzinomen zu finden: 66 Proteine, deren Abwesenheits-/Anwesenheitsmuster der FAP- und sporadischen Krebsgruppierung entsprach können aufgefunden werden (58 ausschließlich in FAP anwesend, 8 nur in sporadischen Karzinomen anwesend). Unter diesen 66 Proteinen konnten vier Proteine identifiziert werden, nämlich ALDH2, KRT18, KRT9 und ECH1, wobei letzteres auch in FAP-Polypen exprimiert wird (vgl. Tabelle 2c). Alle vier Proteine wurden in den FAP-Karzinomen exprimiert, aber nicht in den sporadischen Karzinomen. Diese vier Proteine gruppierten sich innerhalb eines Netzwerks mit einem Punktwert von 12 (Fig. 7). Dieses Netzwerk stand in Beziehung zu Funktionen wie etwa Zelltod, Embryonal-Entwicklung, Zelle-Zelle-Signalisierung und Wechselwirkung.

### Western Blot von normaler Schleimhaut und normalen FAP-Proben

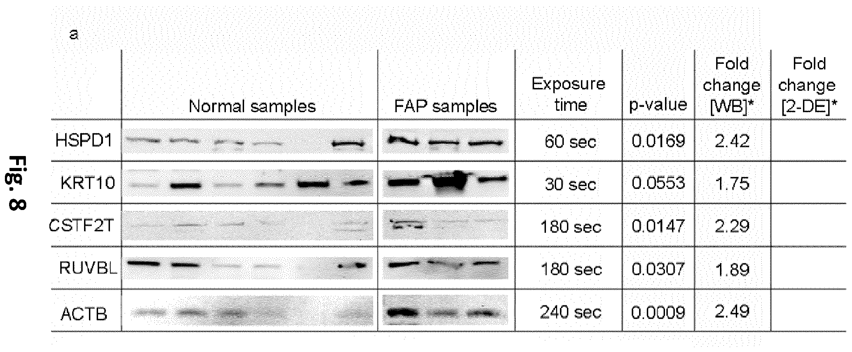

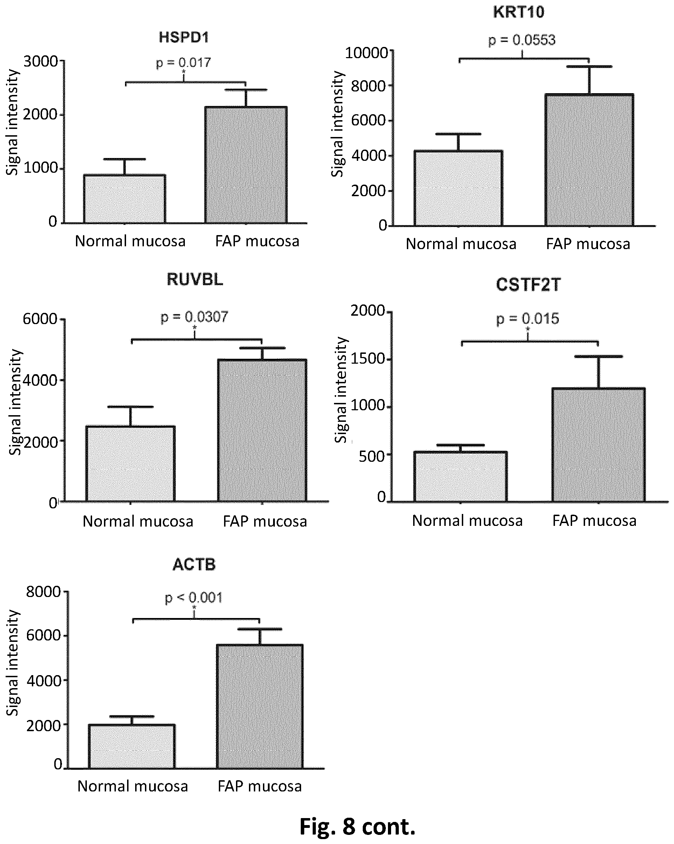

Auf der Basis der IPA-Analyse, Fold-Changes, Molekularfunktionen und Verfügbarkeit von Antikörpern wurden HSPD1, KRT10, CSTF2T, RUVBL, LMNA, und ACTB für die nachgelagerte Analyse durch Western Blot gewählt. Alle diese sechs Proteine besaßen eine höhere Expression bei normalen FAP-Proben relativ zu normalen Schleimhautproben. Eine Western-Blot-Analyse zeigte ein spezifisches Band für KRT10, RUVBL, LMNA, und ACTB, während ein schwaches Mehrfachband für HSPD1 (monoklonaler Antikörper) und CSTF2T (polyklonaler Antikörper) beobachtet werden konnte. Western-Blot-Fold-Changes von allen sechs Proteinen verhielten sich entsprechend der 2-DE-Daten. Vier von sechs Proteinen erreichten dabei eine Signifikanz von p < 0,05. Für KRT10 war der p-Wert marginal größer als 0,05 (p = 0,0553). Eine densitometrische Analyse der Western-Blot-Expression für LMNA erreichte nicht die Signifikanzhöhe (p > 0,05) (Fig. 8).

### Erörterung

Trotz aller Fortschritte in der Medizintechnologie wird eine vererbte Prädisposition für kolorektalen Krebs in vielen Fällen immer noch zu spät diagnostiziert und behandelt, insbesondere bei Patienten ohne offensichtliche Erkrankung in der Familiengeschichte oder wenn genetisches Testen versagt. Das Detektieren der FAP-Krankheit im makroskopisch unbeeinflussten Epithel oder spätestens in einem prämalignen Zustand würde ultimativ zu einer höheren Heilungsrate führen. Die Gelegenheit zum Bestimmen von individuellen Patienten, die wahrscheinlich eine frühe oder noch okkulte Krankheit aufweisen, kann eine überragende Auswirkung auf die zukünftige Diagnose und Therapie der FAP-Krankheit haben.

Das in vorgelegte Konzept zielt darauf ab, ein Muster von differentiell exprimierten Gewebeproteinen zu identifizieren, das es gestattet, eine unbeeinflusste "normale" FAP-Schleimhaut zur frühen Diagnose selbst dann von sporadischer normaler Schleimhaut zu unterscheiden, wenn die Krankheit immer noch okkult ist. Dieses Konzept steht im Kontrast mit allen anderen FAP-Diagnoseansätzen, die auf einzelnen Genmutationen oder individuellen Biomarkern basieren. Mit dem erfindungsgemäßen Konzept wird in erster Linie die diagnostsche Lücke der gegenwärtigen FAP-Diagnose geschlossen.

Nach der 2-DE-basierten Separation von 1950 verschiedenen Polypeptid-Spots in dem Match-Set wird sich auf die ausgeprägtesten Proteinexpressionsänderungen in allen drei Krankheitsstadien konzentriert, das heißt makroskopisch unbeeinflusste "normale" Schleimhaut, Polypen und kolorektale Karzinome. Die Struktur der Probensammlung (verschiedene Krankheitsstadien von dem gleichen Patienten) ermöglicht nicht nur einen Vergleich der Krankheitsexpression zwischen verschiedenen Patienten, sondern auch eine Analyse von normaler Koloanschleimhaut, Polypen und Karzinomen von dem gleichen Patienten. Dies ist vorteilhaft, da die Evaluation weniger durch "ein individuelles Expressionsmuster" beeinflusst ist.

Der Vergleich zwischen makroskopisch unbeeinflusster "normaler" FAP-Schleimhaut und ihrem sporadischen Gegenstück erschien der attraktivste zu sein. Dieser Vergleich könnte das Potential haben, Protein-Biomarker für eine innovative Diagnostik einer okkulten FAP-Krankheit zu identifizieren. Tatsächlich wurden insgesamt 47 Protein-Spots detektiert, die in der normalen FAP-Schleimhaut exprimiert wurden, aber nicht in sporadischer normaler Schleimhaut. Von diesen 47 Protein-Spots wurden 15 Proteine durch Massenspektrometrie identifiziert und 13 von ihnen zu einem Funktionsnetzwerk gruppiert, das zu Zelltod, dermatologischen Krankheiten und Zuständen und Zellfunktion und -erhaltung in Beziehung steht. Die Involvierung der identifizierten, differentiell exprimierten Proteine in den oben genannten Pfaden und Zellfunktionen legte deshalb diese Proteine für eine weitere nachgeschaltete Beurteilung stark nahe. Die sich anschließende Western-Blot-Analyse von sechs ausgewählten Proteinen konnte eine signifikante differentielle Expression für vier von ihnen bestätigen, wobei KRT die Signifikanz marginal verfehlte (p = 0,0553).

Diese Daten legen nahe, dass ein makroskopisch "normales" Epithel eines FAP-Patienten auf der molekularen Ebene bereits einen "FAP-spezifischen Protein-Fingerabdruck" hinterlässt. Interessanterweise sind die meisten der 13 Proteine direkt mit dem Transkriptionsregulator HNF4A und dem Tumorunterdrücker TP53 verbunden und/oder werden von ihnen geregelt. Beide sind wegen ihrer Auswirkung bei der sporadischen kolorektaler Karzinogese wohlbekannt, wurden jedoch bisher nicht beschrieben, dass sie in einer makroskopisch unbeeinflussten "normalen" sporadischen oder FAP-Schleimhaut verändert sind. Innerhalb des Funktionsnetzwerkes der 13 identifizierten Proteine des "FAP-normalen Mucosa-Protein-Fingerabdrucks" regelt TP53 HSPD1 direkt, auch Chaperonin genannt. HSPD1 ist eines der drei Proteine, die nicht nur in FAP-normaler Schleimhaut höher exprimiert werden, sondern auch in FAP-Polypen im Vergleich zu ihrem sporadischen Gegenstück. HSPD1 besitzt eine Signalisierungsfunktion für das angeborene Immunsystem und ist essenziell für das Falten und den Zusammenbau von neu importierten Proteinen in die Mitochondrien. Außerdem gab es Berichte über die verstärkte Expression von HSPD1 in Brust-, Blasen- und Pankreaskarzinomen. Es wurde weiterhin beobachtet, dass Karzinome die HSPD1-Positivität im Vergleich zu Adenomen in CRC verbessern. Weiterhin wird vermutet, dass eine HSPD1-Überexpression in dysplastischen und neoplastischen Epithelzellen auf eine mutierte Form von HSPD1 zurückzuführen sein könnte, das seine funktionale Rolle verloren und sich in dem Zytoplasma akkumuliert hat. Die vorliegenden Daten definieren eine enge Korrelation der HSPD1-Überexpression und der Karzinogese von FAP-Patienten in dem prämalignen Stadium.

Es interagiert auch mit ACTB, das ebenfalls zu dem "FAP-normalen Schleimhautprotein-Fingerabdruck" gehört. ACTB ist ein Hauptbestandteil des kontraktilen Actin-Apparats und eines der beiden nicht-muskel-zytoskeletale Aktine. Es regelt auch CCT5, das ebenfalls ein Teil des "FAP-normalen Schleimhautprotein-Fingerabdrucks" ist und eine Funktion beim Entfalten von Polypeptiden zu verschiedenen Proteinen einschließlich Aktin und Tubulin hat. CCT5 und ACTB wurden so beschrieben, dass sie früh während der Karzinogese an Zellzyklusänderungen beteiligt sind.

Die übrigen zwei Proteine, die nicht nur in FAP-normaler Schleimhaut höher exprimiert sind, sondern auch in FAP-Polypen im Vergleich zu ihrem sporadischen Gegenstück, sind KRT10 und CSTF2T. KRT10 wird in postmitotischen differenzierenden Keratinozyten in der Epidermis in vivo exprimiert, und seine Expression ist in hyperproliferativen Situationen einschließlich Hauttumoren stark reduziert. Die Modulation des Zellwachstums durch Keratin K10 ist verknüpft mit dem Retinoblastoma-Protein (pRB) und der molekularen Maschinerie, die während G1 die Zellzyklus-Progression steuert, insbesondere die Cyclin-D1-Expression. CSTF2T ist ein Teilungsstimulationsfaktor und bisher nicht dafür bekannt, dass er an einer malignen Transformation beteiligt ist.

Die drei Proteine, die in FAP-normaler Schleimhaut und Polypen exprimiert werden, im Vergleich zu den sporadischen Gegenstücken, sind Teil der 49 Proteine, die in den FAP-Polypen exprimiert wurden, aber nicht in den sporadischen Polypen. Von diesen wurden 23 durch Massenspektrometrie identifiziert und es stellte sich heraus, dass sie in zwei funktionalen Netzwerken gruppiert sind, die zu genetischen Störungen, metabolischer Krankheit, Energieproduktion, Zellzyklus, DNA-Replikation, Rekombination und Reparatur in Beziehung standen. Es ist interessant anzumerken, dass HSPD1 und KRT10 in FAP-normaler Schleimhaut und Polypen im Vergleich zu ihren sporadischen Gegenstücken vorliegen und dass sie an allen drei funktionalen Netzwerken der ersten beiden Krankheitsstadiumsvergleiche beteiligt sind (FAP normal versus sporadisch normal und FAP-Polyp versus sporadischer Polyp). Es ist auch bemerkenswert, dass HNF4A als ein zentraler Knoten des funktionalen Netzwerkes "FAP normal versus sporadisch normal" ein zentraler Knoten des Netzwerkes 1 des Vergleichs FAP-Polyp versus sporadischer Polyp ist. HNF4A sollte deshalb als ein Schlüsselprotein bei FAP-bezogener Karzinogese angesehen werden und sollte deshalb bei den weiteren nachgeschalteten Analysen im Brennpunkt stehen. HNFA4 ist ein Mitglied einer Hormonrezeptorfamilie und wird hauptsächlich in Leber und Niere und auf niedrigeren Niveaus in Langerhansschen Inseln, im Dünndarm und im Dickdarm exprimiert. HNF4A regelt die koordinierte Expression von mehreren Genen, die für normale Leber- und Pankreasfunktion erforderlich sind. Es ist ein Schlüsselregulator einer Anzahl von Genen, die beim Glucose-, Cholesterin- und Fettsäuremetabolismus beteiligt sind.

Weiterhin stellte sich heraus, dass ECH1 in FAP-Polypen und FAP-Karzinomen exprimiert wurde, aber nicht in den sporadischen Gegenstücken. ECH1 wird durch TNF reguliert, ein zentraler Knoten des Netzwerks 1 innerhalb des Vergleichs von FAP-Polypen und sporadischen Polypen und des Karzinom-Netzwerks. Deshalb kann angenommen werden, dass TNF ähnlich zu HNF4A ebenfalls eine bisher noch unterschätzte Rolle bei der FAP-assoziierten kolorektalen Karzinogese spielt (vgl. Tabelle 3, in der auch alle klinisch relevanten Proteinmarker zur Früherkennung von FAP aufgeführt sind).

Durch die Beobachtung von Risikofamilien, die Verknüpfungsanalyse und das genetische Testen insbesondere in westlichen Ländern kann der größte Teil von FAP-Patienten entdeckt werden. Die Tatsache jedoch, dass wir drei Krebsproben sammeln konnten, spiegelt das diagnostische Dilemma und die Notwendigkeit für empfindlichere Tests wider. Diese diagnostische Lücke kann dadurch erklärt werden, dass etwa 22 % bis 46 % von FAP auf "de novo" Mutationen ohne assoziierte Familiengeschichte basieren. Eine nicht-subjektive Klassifikation von bösartigen Tumoren und die Aufklärung von durch genomische oder proteomische Profilierung entdeckten biologischen Markern wurde in mehreren Publikationen berichtet. In Experimenten, die im Rahmen der vorliegenden Erfindung durchgeführt wurden, gelang es einen in den linken Eierstock metastasierten, schlecht differenzierten sigmoidalen Dickdarmkrebs durch eine 2-DE-Expressionsanalyse in Kombination mit einer multivariaten Statistik von einem primären Eierstockkrebs zu unterscheiden. Bei einer genomischen, proteomischen und Gewebe-Array-Studie an 60 menschlichen Krebszelllinien wurden zuvor die Proteine Villin und Moezin identifiziert und als Marker vorgeschlagen, um zwischen primären bösartigen Eierstock- und Dickdarmtumoren zu differenzieren. So ist man bereits in der Lage, verschiedene Krankheitsstadien bei Prostata-, Brust- und Eierstockkrebs auf der Basis von krankheits- und stadienspezifischen Proteinprofilen zu trennen. Das Durchsuchen der veröffentlichten Listen von potentiellen Markerproteinen offenbart wiederholt auftretende Moleküle, die in dem normalen und dem Krankheitsstadium differentiell exprimiert werden. Mehrere von diesen (zum Beispiel HSPs, Vimentin, Villin usw.) scheinen zu bösartigen Tumoren in Beziehung zu stehen, aber nicht spezifisch für eine bestimmte Tumorentität. Von dem größten Teil der Proteomik-basierten Studien abgeleitete Daten legen nahe, dass "der einzelne Marker" für eine Krebsentität wahrscheinlich nicht existiert. Krebs ist eine Krankheit von gestörten normalen Zellen, und die malignen Zellen werden mit größter Wahrscheinlichkeit nicht die Produktion eines im Körper einmaligen neuen Polypeptids beginnen. Somit erscheint es realistisch, dass die Anwesenheit von Krebs durch multiplexierte Testplatten detektiert wird, die Proteine messen, die als eine Konsequenz einer aberranten Zellfunktion und Zellinteraktionen in Gewebe- und/oder Serumproben produziert werden.

Die in der vorliegenden Anmeldung ausgewählten Polypeptide konnten FAP versus sporadische normale Schleimhaut und Polypen in 100 % der untersuchten Fälle klassifizieren. Dies impliziert, dass jene Proteine das Potenzial besitzen, als eine Gruppe von biologischen Markern zum Entdecken von Erbkrankheiten und ihrem beginnenden Einsetzen bereits auf gutartigen Niveaus verwendet werden zu können. Die in Polypen und Krebs dysregulierten Proteine können neue Informationen hinsichtlich der Tumorbiologie von familiäre adenomatösen Polyposis Coli im Vergleich zu der nicht erblichen Krankheit liefern. Zu berücksichtigen ist, dass Validierungsstudien an klinischen Proben, die von gut kontrollierten Patientenpopulationen gewonnen wurden (zum Beispiel jungen, noch unbeeinflussten FAP-Risiko-Patienten) durchgeführt werden sollten, bevor jene Marker für eine Routinediagnose, Prognose oder therapeutische Entscheidung in Betracht gezogen werden können.

Die hier beschriebenen Proteinmarker haben das Potenzial für ein Panel von Antikörpern zu offenbaren, die zum Beispiel in einer Chip-Oberfläche integriert oder in einem ELISA-Test verwendet werden.

Die erhaltenen Daten zeigen spezifische Differenzen von FAP- und sporadischer kolorektalen Krankheit auf dem Proteinexpressionslevel und könnten Patienten mit FAP-Krankheit bereits in makroskopisch "normaler" kolorektaler Schleimhaut identifizieren helfen.

**Tabelle 1: Gewebeproben und klinische Daten**

| **Gewebe** | | | | **Patient** | | |
|---|---|---|---|---|---|---|
| **Nr** | **Normal** | **Adenom** | **Tumor** | **Geschlecht** | **Alter** | **TNM** |
| ***Sporadisch auftretendes Karzinom*** | | | | | | |
| 1 | CR107N | CR105P | CR106T | F | 71 | T3N0Mx |
| 2 | CR135N | CR133P | CR134T | M | 77 | T4N1Mx |
| 3 | CR139N | CR138P | CR137T1, CR136T2 | M | 78 | T4N1Mx |
| 4 | CR149N | CR150P | CR148T | M | 60 | T3N0Mx |
| 5 | CR164N | CR162P1, CR163P2 | CR161T | M | 61 | T3N0Mx |
| 6 | CR174N | CR173P | CR172T | M | 74 | T3N1Mx |
| 7 | CR211N | CR210P | CR209T | M | 76 | T3N1Mx |

| ***FAP*** | | | | | | |
|---|---|---|---|---|---|---|
| 1 | FAP1N | FAP1P | | M | 30 | |
| 2 | FAP2N | FAP2P1, FAP2P2 | | F | 32 | |
| 3 | FAP3N | FAP3P1, FAP3P2 | | M | 34 | |
| 4 | FAP5N | FAP5P1, FAP5P2, FAP5P3 | | F | 22 | |
| 5 | FAP6N | FAP6P1, FAP6P2, FAP6P3 | | M | 36 | |
| 6 | FAP7N | FAP7P | | M | 31 | |
| 7 | FAP8N | FAP8P | FAP8T1, FAP8T2 | M | 45 | T3N0Mx, T3N0Mx |
| 8 | FAP9N | FAP9P | FAP9T | F | 29 | T3N0Mx |

| | | | | | | |
|---|---|---|---|---|---|---|
| FAP: Familiäre adenomatöse Polyposis; N: normale Mucosa; P: Polyp; T: Karzinom; F: weiblich; M: männlich; TNM: Klassifikation gemäß WHO-Kriterien. | | | | | | |

**Tabelle 2a: Stärker exprimierte Proteine in makroskopisch nicht betroffenen "normalen" FAP Mucosa-Zellen**

| **Spot** | **Mittel in FAP normal** | **Variationskoeffizient** | **Accession Nr** | **Symbol** | **Name** | **SequenzAbdeckung [%]** | **Treffer/ gesuchte Peptide** | **Genomkontext** | **IPA Netzwerk / Treffer** |
|---|---|---|---|---|---|---|---|---|---|
| **3322** | 4.02 | 0.25 | AAH54049 | **RNF115** | Ring finger protein 115 | 16 | 3/45 | 1q21.1 | 1 / 37 |
| **3722** | 4.86 | 0.29 | AAH16179 | **HSPA8** | HSP70 protein 8 | 10 | 4/52 | 11q24.1 | 1 / 37 |
| **4622** | 4.89 | 0.3 | AAA36022 | **HSPD1*** | HSP60 (Chaperonin) | 13 | 5/48 | 2q33.1 | 1 / 37 |
| **4725** | 5.79 | 0.14 | NP_937831 | **ASTN2** | Astrotactin 2 isoform d | 15 | 4/57 | 9q33.1 | ungeeignet |
| **4830** | 5.36 | 0.32 | CAA52924 | **KRT9** | Keratin 9 | 32 | 11/64 | 17q21 | 1 / 37 |
| **5448** | 3.9 | 0.31 | | | | 19 | 6/55 | | |
| **7443** | 4.79 | 0.19 | | | | 46 | 17/66 | | |
| **5122** | 3.33 | 0.45 | AAA60544 | **KRT10 *** | Keratin 10 | 13 | 3/44 | 17q21 | 1 / 37 |
| **5529** | 4.89 | 0.16 | NP_003356 | **UQCRC 1** | Ubiquinolcytochrome c reductase core protein I | 27 | 8/47 | 3p21.3 | 1 / 37 |
| **5630** | 5.62 | 0.13 | NP_036205 | **CCT5** | Chaperonin containing TCP 1. subunit 5 (epsilon) | 25 | 9/57 | 5p15.2 | 1 / 37 |
| **5708** | 4.31 | 0.13 | AAH30833 | **NDUFS1** | NADH dehydrogenase Fe-S protein I | 10 | 5/63 | 2q33-q34 | 1 / 37 |
| **6562** | 5.36 | 0.15 | AAH12854 | **ACTB** | Actin, beta | 35 | 8/66 | 7p15-p12 | 1 / 37 |
| **6756** | 5.88 | 0.2 | CAI15527 | **LMNA** | Lamin A/C | 24 | 9/54 | 1q21 | 1 / 37 |
| **7558** | 5.39 | 0.14 | NP_000657 | **ACY1** | Aminoacylase 1 | 28 | 9/46 | 3p21.1 | 1 / 37 |
| **8523** | 6.14 | 0.18 | NP_003698 | **RUVBL 1** | RuvB-like 1 | 22 | 6/58 | 3q21 | 1 / 37 |
| **8634** | 6.57 | 0.11 | AAH28239 | **CSTF2T *** | Cleavage stimulation factor | 16 | 5/69 | 10q11 | 1 / 37 |
| **8644** | 5.02 | 0.19 | AAH69229 | **TIGD5** | tigger transposable element derived 5 | 13 | 5/66 | 8q24.3 | ungeeignet |

| | | | | | | | | | |
|---|---|---|---|---|---|---|---|---|---|
| Mittel = durchschnittlicher log Wert über FAP für das Protein; Spot: SSP Spotnummer; IPA = Ingenuity Pathway Analysis; hpr = Human Protein Atlas (www.proteinatlas.org); n.a. = nicht verfügbar; n = Nummer * auch in FAP Polypen exprimiert; | | | | | | | | | |

**Tabelle 2b: In FAP Polypen stärker exprimierte Proteine**

| **Spot** | **Mittel in FAP polyp** | **Variationskoeffizient** | **Accession Nr** | **Symbol** | **Name** | **SequenzAbdeckung [%]** | **Treffer/ gesuchte Peptide** | **Genomkontext** | **IPA Netzwerk / Treffer** |
|---|---|---|---|---|---|---|---|---|---|
| **1254** | 6.08 | 0.10 | AAH34697 | **KRT10*** | Keratin 10 | 18 | 6/76 | 17q21 | 1 / 37 |
| **3342** | 5.58 | 0.19 | NP_776959 | **DNASE1** | Deoxyribonuklease I | | | | |
| **3620** | 6.17 | 0.21 | NP_005564 | **LMNB1** | Lamin B1 | 46 | 23/69 | 5q23.3-q31.1 | |
| **3678** | 6.52 | 0.10 | NP_006073 | **TU-BA1B** | Tubulin, alpha 1b | 43 | 15/67 | 12q13.12 | |
| **4254** | 4.98 | 0.11 | CAA52924 | **KRT9** | Keratin 9 | 29 | 11/67 | 17q21.1-q21.2 | |
| **4257** | 5.65 | 0.21 | NP_009294 | **COMT** | Katechol-O-methyltransferase | 44 | 6/42 | 11q23 | |
| **4622** | 5.08 | 0.28 | AAA36022 | **HSPD1*** | HSP60 (Chaperonin) | 13 | 5/48 | 2q33.1 | 1 / 37 |
| **4652** | 5.74 | 0.17 | NP_002083 | **GRSF1** | G-rich RNA Sequence binding factor 1 | 17 | 7/60 | 4q13 | |
| **5258** | 5.87 | 0.14 | CAG46492 | **RAB11B** | RAB 11B. member RAS Onkogen Familie | 27 | 6/37 | 19p13.2 | |
| **5414** | 6.18 | 0.07 | AAC31758 | **CKB** | Kreatinkinase | 19 | 5/51 | 14q32 | |
| **6359** | 6.19 | 0.12 | NP_001144 | **ANXA4** | Annexin A4 | 35 | 9/46 | 2p13 | |
| **6421** | 5.89 | 0.11 | AAH66325 | **THOC3** | THO Komplex 3 | 25 | 6/61 | 5q35.2 | |
| **6445** | 7.13 | 0.13 | NP_005521 | **IDH3A** | Isocitrate dehydrogenase 3 (NAD+) alpha | 30 | 12/34 | 15q25.1-q25.2 | |
| **6656** | 6.18 | 0.12 | NP_001924 | **DLST** | Dihydrolipoamid S-succinyltransferase | 20 | 10/40 | 14q24.3 | |
| **7460** | 4.96 | 0.29 | NP_000008 | **ACAD5** | Acyl-Coenzyme A dehydrogenase | 17 | 6/33 | 19p13.2 | |
| **7539** | 5.01 | 0.24 | AAA35590 | **BCK-DHA** | Branched chain keto acid dehydrogenase E1 | 53 | 14/63 | 19q13.1-q13.2 | |
| **7544** | 4.52 | 0.23 | NP_001147 | **ANXA7** | Annexin A7 | 27 | 12/49 | 10q21.1-q21.2 | |
| **7561** | 6.61 | 0.08 | NP_002970 | **SCP2** | Sterol carrier protein 2 | 18 | 7/72 | 1p32 | |
| **7613** | 6.38 | 0.17 | CAI42100 | **POF1B** | Premature ovarian failure. 1B | 14 | 8/46 | Xq21.1-q21.2 | |
| **8305** | 5.21 | 0.16 | P53701 | **HCCS** | Holocytochrome c synthase | 17 | 5/54 | Xp22.3 | |
| **8324** | 4.84 | 0.43 | AAA35730 | **CYC1** | Cytochrome c-1 | 40 | 6/58 | 8q24.3 | |
| **8344** | 4.72 | 0.3 | AAH17408 | **ECH1**** | Enoyl Coenzyme A hydratase 1 | 16 | 5/51 | 19q13.1 | |
| **8634** | 6.38 | 0.11 | AAH28239 | **CSTF2T** * | Cleavage stimulation factor | 16 | 5/69 | 10q11 | |

| | | | | | | | | | |
|---|---|---|---|---|---|---|---|---|---|
| * auch in FAP normalen Mukosazellen exprimiert; ** auch in FAP Karzinomzellen exprimiert. | | | | | | | | | |

**Tabelle 2c: In FAP Karzinomzellen stärker exprimierte Proteine**

| **Spot** | **Mittel in FAP polyp** | **Variationskoeffizient** | **Accession Nr** | **Symbol** | **Name** | **SequenzAbdeckung [%]** | **Treffer/ gesuchte Peptide** | **Genomkontext** | **IPA Netzwerk** / **Treffer** |
|---|---|---|---|---|---|---|---|---|---|
| **7676** | 1.88 | 0.02 | AAT41621 | **ALDH2** | Aldehyddehydrogenase 2 Familie (mitoch.) | 11 | 4/75 | 12q24.2 | |
| **1450** | 1.8 | 0.18 | AAH00698 | **KRT18** | Keratin 18 | 17 | 6/42 | 12q13 | |
| **3029** | 1.76 | 0.1 | CAA52924 | **KRT9** | Keratin 9 | 15 | 5/47 | 17q21.1-q21.2 | |
| **6657** | 1.67 | 0 | AAT41621 | **ALDH2** | Aldehyddehydrogenase 2 Familie (mitoch.) | 14 | 5/86 | 12q24.2 | |
| **8344** | 1.63 | 0.27 | AAH17408 | **ECH1**** | Enoyl Coenzym A hydratase 1 | 16 | 5/51 | 19q13.1 | |
| **1161** | -0.21 | -9.47 | CAA52924 | **KRT9** | Keratin 9 | 12 | 4/46 | 17q21.1-q21.2 | |

| | | | | | | | | | |
|---|---|---|---|---|---|---|---|---|---|
| ** auch in FAP Polypen exprimiert | | | | | | | | | |

**Tabelle 3: Protein zur klinischen Anwendung beim Erkennen von FAP in Makroskopisch normaler Colon-Mukosa**

| **Spot** | **Mittel in FAP polyp** | **Variationskoeffizient** | **Accession Nr.** | **Symbol** | **Name** | **signifikant bei Normal** | **signifikant bei Polypen** | **signifikant bei Krebs** | **Western Blot** | **Genomic context** |
|---|---|---|---|---|---|---|---|---|---|---|
| **3322** | 4.02 | 0.25 | AAH54049 | **RNF115** | Ring finger protein 115 | X | | | | 1q21.1 |
| **3722** | 4.86 | 0.29 | AAH16179 | **HSPA8** | HSP70 protein 8 | X | | | | 11q24.1 |
| **4622** | 4.89 | 0.3 | AAA36022 | **HSPD1** | HSP60 (Chaperonin) | X | X | | **ja** | 2q33.1 |
| **4725** | 5.79 | 0.14 | NP_937831 | **ASTN2** | Astrotactin 2 isoform d | X | | | | 9q33.1 |
| **4830** | 5.36 | 0.32 | CAA52924 | **KRT9** | Keratin 9 | X | | | | 17q21 |
| **5448** | 3.9 | 0.31 | | | | | | | | |
| **7443** | 4.79 | 0.19 | | | | | | | | |
| **5122** | 3.33 | 0.45 | AAA60544 | **KRT10** | Keratin 10 | X | X | | **ja** | 17q21 |
| **5529** | 4.89 | 0.16 | NP_003356 | **UQCRC 1** | Ubiquinolcytochrome c reductase core protein I | X | | | | 3p21.3 |
| **5630** | 5.62 | 0.13 | NP_036205 | **CCT5** | Chaperonin containing TCP1. subunit 5 (epsilon) | X | | | | 5p15.2 |
| **5708** | 4.31 | 0.13 | AAH30833 | **NDUFS1** | NADH dehydrogenase Fe-S Protein I | X | | | | 2q33-q34 |
| **6562** | 5.36 | 0.15 | AAH12854 | **ACTB** | Altin, beta | X | | | **ja** | 7p15-p12 |
| **6756** | 5.88 | 0.2 | CAI15527 | **LMNA** | Lamin A/C | X | | | | 1q21 |
| **7558** | 5.39 | 0.14 | NP_000657 | **ACY1** | Aminoacylase 1 | X | | | | 3p21.1 |
| **8523** | 6.14 | 0.18 | NP_003698 | **RUVBL 1** | RuvB-like 1 | X | | | **ja** | 3q21 |
| **8634** | 6.57 | 0.11 | AAH28239 | **CSTF2T** | Cleavage stimulation factor | X | X | | **ja** | 10q11 |
| **8644** | 5.02 | 0.19 | AAH69229 | **TIGD5** | tigger transposable element derived 5 | X | | | | 8q24.3 |
| **8344** | 1.63 | 0.27 | AAH17408 | **ECH1** | Enoyl Coenzym A hydratase 1 | | X | X | | 19q13.1 |
| **7676** | 1.88 | 0.02 | AAT41621 | **ALDH2** | Aldehyddehydrogenase 2 Familie (mitoch.) | | | X | | 12q24.2 |
| **1450** | 1.8 | 0.18 | AAH00698 | **KRT18** | Keratin 18 | | | X | | 12q13 |
| Zentrales Protein bei FAP assoziierter Karzinogenese basierend auf IPA | | | P41235 | **HNF4A** | Hepatocyte nuclear factor 4, alpha | | | | | 20q13.12 |
| Zentrales Protein bei FAP assoziierter Karzinogenese basierend auf IPA | | | P01375 | **TNF** | Tumor necrosis factor | | | | | 6p21.3 |

| | | | | | | | | | | |
|---|---|---|---|---|---|---|---|---|---|---|
| Mittel = durchschnittlicher log Wert über FAP für das Protein; Spot = SSP Spotnummer; IPA = Ingenuity Pathway Analyse; hpr = Human Protein Atlas (www.proteinatlas.org). | | | | | | | | | | |

## Patentansprüche

1. Verfahren zum Diagnostizieren der familiären adenomatösen Polyposis (FAP), **gekennzeichnet durch** die Schritte:
Bestimmen der Expression wenigstens eines Proteins ausgewählt aus der Gruppe bestehend aus RNF115 (Ring Finger Protein 115), HSPA8 (HSP70 Protein 8), HSPD1 (HSP60 Chaperonin), ASTN2 (Astrotactin 2 Isoform d), KRT9 (Keratin 9), KRT10 (Keratin 10), UQCRC1 (Ubiquinol-Cytochrom c Reduktase Core Protein I), CCT5 (Chaperonin containing TCP1 Subunit 5 (epsilon)), NDUFS1 (NADH Dehydrogenase Fe-S Protein I), ACTB (Actin, beta), LMNA (Lamin A/C), ACY1 (Aminoacylase 1),
RUVBL1 (RuvB-like 1), CSTF2T (Cleavage Stimulation Factor), TIGD5 (Tigger Transposable Element derived 5), ECH1 (Enoyl Coenzyme A Hydratase 1), ALDH2 (Aldehyd-Dehydrogenase 2 Familie (mitoch.)) und KRT18 (Keratin 18) in einer Gewebeprobe eines ersten Individuums; und
Vergleichen der Expression des wenigstens einen Proteins in der Gewebeprobe des ersten Individuums mit der Expression des wenigstens einen Proteins in einer Gewebeprobe eines gesunden zweiten Individuums und/oder eines dritten Individuums mit sporadisch kolorektalem Karzinom,
wobei das Vorliegen einer erhöhten Expression des wenigstens einen Proteins in der Gewebeprobe des ersten Individuums das Vorliegen der familiären adenomatösen Polyposis (FAP) anzeigt, und wobei die Gewebeprobe eine Probe der Darmschleimhaut ist;
**dadurch gekennzeichnet, dass** das Vorliegen einer erhöhten Expression von ECH1 in einem Polypen und/oder Krebszellen des ersten Individuums das Vorliegen der familiären adenomatösen Polyposis (FAP) anzeigt; und
das Vorliegen einer erhöhten Expression von ALDH2 und/oder KRT18 in Krebszellen des ersten Individuums das Vorliegen der familiären adenomatösen Polyposis (FAP) anzeigt.

2. Verfahren nach Anspruch 1, **dadurch gekennzeichnet, dass** zusätzlich die Expression von HNF4A und/oder TNF in der Gewebeprobe des ersten Individuums bestimmt und mit der Expression von HNF4A und/oder TNF in der Gewebeprobe des zweiten Individuums und/oder der Gewebeprobe des dritten Individuums verglichen wird, wobei das Vorliegen einer erhöhten Expression von HNF4A und/oder TNF in der Gewebeprobe des ersten Individuums das Vorliegen der familiären adenomatösen Polyposis (FAP) anzeigt.

3. Verwendung eines Kits in dem Verfahren nach Anspruch 1 oder 2, wobei das Kit Antikörper umfasst, die spezifisch für den Nachweis von mindestens einem Protein ausgewählt aus einer Gruppe bestehend aus RNF115, HSPA8, HSPD1, ASTN2, KRT9, KRT10, UQCRC1, CCT5, NDUFS1, ACTB, LMNA, ACY1, RUVBL1, CSTF2T, TIGD5, ECH1, ALDH2, KRT18 und optional FAP, HNF4A und/oder TNF Proteine sind.

4. Verwendung einer multiplexen Testplatte in dem Verfahren nach Anspruch 1 oder 2, auf der Antikörper aufgebracht sind, die spezifisch den Nachweis von mindestens einem Protein erlauben, ausgewählt aus einer Gruppe bestehend aus RNF115, HSPA8, HSPD1, ASTN2, KRT9, KRT10, UQCRC1, CCT5, NDUFS1, ACTB, LMNA, ACY1, RUVBL1, CSTF2T, TIGD5, ECH1, ALDH2, KRT18 und optional FAP, HNF4A und/oder TNF.

5. Verwendung nach Anspruch 4, wobei die multiplexe Testplatte eine Chip-Oberfläche und/oder ein ELISA ist.

## Claims

1. Method for diagnosing familial adenomatous polyposis (FAP), **characterized by** the steps:
determining the expression of at least one protein selected from the group consisting of RNF 115 (ring finger protein 115), HSPA8 (HSP70 protein 8), HSPD1 (HSP60 chaperonin), ASTN2 (astrotactin 2 isoform d), KRT9 (keratin 9), KRT10 (keratin 10), UQCRC1 (Ubiquinol-cytochrom c reductase core protein I), CCT5 (chaperonin containing TCP1 subunit 5 (epsilon)), NDUFS1 (NADH dehydrogenase Fe-S protein I), ACTB (actin, beta), LMNA (lamin A/C), ACY1 (aminoacylase 1),
RUVBL1 (RuvB-like 1), CSTF2T (cleavage stimulation factor), TIGD5 (tigger transposable element derived 5), ECH1 (Enoyl coenzyme A hydratase 1), ALDH2 (aldehyd-dehydrogenase 2 family (mitoch.)) and KRT18 (keratin 18) in a tissue sample of a first individual; and
comparing the expression of the at least one protein in a tissue sample of the first individual with the expression of the at least one protein in a tissue sample of a healthy second individual and/or of a third individual with sporadic colorectal carcinoma,
wherein the presence of an increased expression of the at least one protein in the tissue sample of the first individual indicates the presence of familial adenomatous polyposis (FAP), and wherein the tissue sample is a sample of the intestinal mucosa; **characterized in that** the presence of an increased expression of ECH1 in a polyp and/or cancer cells of the first individual indicates the presence of familial adenomatous polyposis (FAP); and
the presence of an increased expression of ALDH2 and/or KRT18 in cancer cells of the first individual indicates the presence of familial adenomatous polyposis (FAP).

2. Method according to claim 1, **characterized in that** additionally the expression of HNF4A and/or TNF in the tissue sample of the first individual is determined and compared to the expression of HNF4A and/or TNF in the tissue sample of the second individual and/or the tissue sample of the third individual, wherein the presence of an increased expression of HNF4A and/or TNF in the tissue sample of the first individual indicates the presence of familial adenomatous polyposis (FAP).

3. Use of a kit in the method according to claim 1 or 2, wherein the kit comprises antibodies specific for the detection of at least one protein selected from the group consisting of RNF115, HSPA8, HSPD1, ASTN2, KRT9, KRT10, UQCRC1, CCT5, NDUFS1, ACTB, LMNA, ACY1, RUVBL1, CSTF2T, TIGD5, ECH1, ALDH2, KRT18 and optionally FAP, HNF4A and/or TNF proteins.

4. Use of a multiplex test plate in the method according to claim 1 or 2 onto which antibodies are set out that specifically enable the detection of at least one protein selected from the group consisting of RNF115, HSPA8, HSPD1, ASTN2, KRT9, KRT10, UQCRC1, CCT5, NDUFS1, ACTB, LMNA, ACY1, RUVBL1, CSTF2T, TIGD5, ECH1, ALDH2, KRT18 and optionally FAP, HNF4A and/or TNF.

5. Use according to claim 4, wherein the multiplex test plate is a chip surface and/or ELISA.

## Revendications

1. Procédé pour diagnostiquer la polypose adénomateuse familiale (FAP), **caractérisé par** les étapes:
détermination de l'expression d'au moins une protéine choisie dans le groupe consistant en RNF115 (Ring Finger Protein 115), HSPA8 (HSP70 Protein 8), HSPD1 (HSP60 Chaperonin), ASTN2 (Astrotactin 2 Isoform d), KRT9 (Keratin 9), KRT10 (Keratin 10), UQCRC1 (Ubiquinol-Cytochrom c Reductase Core Protein I), CCT5 (Chaperonin containing TCP1 Subunit 5 (epsilon)), NDUFS1 (NADH Dehydrogenase Fe-S Protein I), ACTB (Actin, bêta), LMNA (Lamin A/C), ACY1 (Aminoacylase 1), RUVBL1 (RuvB-like 1), CSTF2T (Cleavage Stimulation Factor), TIGD5 (Tigger Transposable Element derived 5), ECH1 (Enoyl Coenzyme A Hydratase 1), ALDH2 (famille de Aldehyde-Dehydrogenase 2 (mitoch.)) et KRT18 (Keratin 18) dans un échantillon de tissu d'un premier individu; et
comparaison de l'expression de la au moins une protéine dans l'échantillon de tissu du premier individu avec l'expression de la au moins une protéine dans un échantillon de tissu d'un deuxième individu en bonne santé et/ou d'un troisième individu ayant un carcinome colorectal sporadique,
où la présence d'une expression accrue de la au moins une protéine dans l'échantillon de tissu du premier individu indique la présence de polypose adénomateuse familiale (FAP), et où l'échantillon de tissu est un échantillon de muqueuse intestinale;
**caractérisé en ce que** la présence d'une expression accrue de ECH1 dans un polype et/ou des cellules cancéreuses du premier individu indique la présence de polypose adénomateuse familiale (FAP); et
la présence d'une expression accrue de ALDH2 et/ou KRT18 dans des cellules cancéreuses du premier individu indique la présence de polypose adénomateuse familiale (FAP).

2. Procédé selon la revendication 1, **caractérisé en ce que**, en outre, l'expression de HNF4A et/ou TNF dans l'échantillon de tissu du premier individu est déterminée et comparée avec l'expression de HNF4A et/ou TNF dans l'échantillon de tissu du deuxième individu et/ou l'échantillon de tissu du troisième individu, où la présence d'une expression accrue de HNF4A et/ou TNF dans l'échantillon de tissu du premier individu indique la présence de polypose adénomateuse familiale (FAP).

3. Utilisation d'un kit dans le procédé selon la revendication 1 ou 2, où le kit comprend des anticorps qui sont spécifiques pour la mise en évidence d'au moins une protéine choisie dans un groupe consistant en les protéines RNF115, HSPA8, HSPD1, ASTN2, KRT9, KRT10, UQCRC1, CCT5, NDUFS1, ACTB, LMNA, ACY1, RUVBL1, CSTF2T, TIGD5, ECH1, ALDH2, KRT18 et éventuellement FAP, HNF4A et/ou TNF.

4. Utilisation d'une plaque de test multiplex dans le procédé selon la revendication 1 ou 2, sur laquelle sont appliqués des anticorps qui permettent spécifiquement la mise en évidence d'au moins une protéine choisie dans un groupe consistant en RNF115, HSPA8, HSPD1, ASTN2, KRT9, KRT10, UQCRC1, CCT5, NDUFS1, ACTB, LMNA, ACY1, RUVBL1, CSTF2T, TIGD5, ECH1, ALDH2, KRT18 et éventuellement FAP, HNF4A et/ou TNF.

5. Utilisation selon la revendication 4 où la plaque de test multiplex est une surface de puce et/ou un ELISA.
